# EUROPEAN PATENT SPECIFICATION

(11) **EP 2 461 680 B1**
(45) Date of publication and mention of the grant of the patent: **10.02.2016**
(21) Application number: 10794779.8
(22) Date of filing: 01.07.2010
(51) Int. Cl.: A01N 37/18, A61K 38/00

(54) **NUTRITIONAL SUPPLEMENT**
NÄHRSTOFFERGÄNZUNG
COMPLÉMENT NUTRITIONNEL

(30) Priority: 02.07.2009 US 222735 P
(43) Date of publication of application: 13.06.2012
(73) Proprietor: Keraplast Technologies, Ltd., San Antonio, TX 78258-3103 (US)
(72) Inventor: KELLY, Robert, James McClelland, 8022 Christchurch (NZ); MARSH, Clive, RD6 Christchurch (NZ)
(74) Representative: Jones, Nicholas Andrew
(86) International application number: PCT/US2010/040813
(87) International publication number: WO 2011/003015

(56) References cited:
- WO-A1-03/006531
- US-A- 3 970 614
- US-A- 5 276 138
- US-A1- 2006 280 840
- US-A1- 2007 141 230
- US-A1- 2008 089 930
- BUCHANAN, J. H. ET AL.: 'A Cystine-Rich Protein Fraction from Oxidized alpha-Keratin.' BIOCHEMICAL JOURNAL vol. 167, November 1977, pages 489 - 491, XP008150914

## Description

### Background of The Invention

High protein nutrition sources are important for parts of the sports nutrition market as well as for providing a balanced food supplement to undernourished people. Sulfur and its derivatives, such as cystine and glutathione, are very important in influencing the redox balance in the body and influencing cell-based processes and related overall health. Keratin sources, such as feathers, horns, hooves and wool, are very high in protein and in sulfur amino acids, but are typically of low digestibility and nutritional value due to the high degree of crosslinking associated with the cystine bonding present in keratins.

One method of making human nutritional products from keratin sources such as feathers is described in U.S. Patent No. 3,970,614. The described process includes solubilizing whole feathers or other keratin source in dimethyl formamide. Feathers are solubilized by boiling in 75-100% dimethyl formamide at ambient pressure. The resulting protein gel is precipitated and the DMF recovered by distillation.

Other conventional methods to make keratins more digestible involve the use of hydrolysis to break down large protein chains and modify cystine bonds. The resulting peptides are characterized by strong off tastes and odors and so are not palatable as human nutrition sources. Certain oxidation processes, for example, have been used to increase the solubility of keratin proteins and peptides by oxidizing sulfur containing amino acids such as cysteine to introduce increased negative charge to the molecules.

A process for producing soluble keratin protein products from wool is described in U.S. Patent No. 5,276,138. This patent states, however, that "if the pH of the liquid medium is less than 7, the oxidizing agents do not function well" (column 3, row 19). It further states that high concentrations (>20%) of oxidizing agent are needed, and specifically that "when the concentration of the oxidizing agents are less than 10% an expected object of the present invention cannot be achieved." Although the '138 patent states that the product could be used as a food additive, the product as described would not be expected to be palatable to humans.

### Summary

The present disclosure provides novel protein compositions that are suitable for human consumption and are obtained from insoluble keratin sources such as wool, animal hair, or feathers, for example. The present disclosure arises from the discovery that a soluble keratin protein product can be produced by treating an insoluble keratin source in an oxidizing solution at low pH and heat for a sufficient time to oxidize essentially all of the cysteine residues in the protein. By then raising the pH to the pKa of the proteins, or to the point at which where the protein is present in the salt form, a solution of high molecular weight proteins that are in a low salt solution can be obtained. This solution is essentially free of peptides and salts that have an adverse effect on flavor and palatability and maintains a high proportion of sulfur amino acids for beneficial health effects.

The solution can be further refined if desired by neutralizing any residual oxidant and by various filtration techniques. The resulting protein product can be dried and milled to obtain a powdered product that is suitable for dissolving into water, juice, or a nutritional drink, or for sprinkling on food. Alternatively, the protein can be provided as a liquid supplement, or as a concentrate for addition to water, juice or other drinks such as flavored drinks. Any of the nutritional supplements can further include additional nutrients, vitamins, minerals, anti-oxidants, colorants, flavorants, sweeteners, thickeners, preservatives, or other approved food additives.

The disclosed protein compositions are suitable for protein supplementation for any purpose including dietary supplements for infants, the aged, for athletes, for those seeking weight gain, for those recovering from illness or injury where a readily digestible protein liquid is desirable, such as when recovering from chemotherapy, or for anyone seeking a high protein or sulfur boosted diet. The protein compositions can also be provided as tablets, drinks, hot or cold teas or incorporated into bars.

The present disclosure includes, therefore, soluble keratin protein compositions either in powder or liquid form as well as novel methods of producing soluble keratin proteins by oxidation.

### Detailed Description

The present disclosure includes water soluble keratin protein compositions useful as nutritional supplements that are produced from insoluble sources of keratin such as wool, horns, hooves, animal hair, and feathers, and methods of producing the protein compositions. It is an aspect of the disclosure that keratin protein powders that are suitable for human consumption are produced through oxidation of an insoluble keratin source at low pH. It is a further aspect of the disclosure that a nutrition source is produced using avian feathers as the keratin source. The disclosed processes are effective to cleave the disulfide bonds that are characteristic of keratin proteins and that contribute to the structure and insolubility of the keratin sources. Cleavage of these bonds as described herein produces a protein composition that is soluble, of palatable taste and odour and digestible.

In the present disclosure, the first step of oxidation is undertaken with a low concentration of an oxidant. By low concentration of oxidant is meant an oxidant concentration of from 1-10%, inclusive, and can be 2%, 3%, 4%, 5%, 6%, 7%, 8%, 9%, or any fraction of the integers between 1 and 10%. A variety of oxidants can be used in the described processes, with performic acid, peracetic acid or hydrogen peroxide being exemplary, without limiting the described processes to those particular oxidants. It is a novel aspect of the disclosure that no alkali is added during the oxidation process. As a result of this the oxidation step is acidic, and the pH of the mixture following oxidation is typically from 1.5-5 and most commonly the pH is 3.6. In one embodiment of the invention acid is added during the oxidation step, and the pH maintained around 1.5-3. Various mineral or organic acids such as sulfuric acid, for example, can be used in the process. To ensure complete oxidation, the oxidation step can be practiced at elevated temperatures for extended times. In certain embodiments, the oxidation step is maintained at an elevated temperature such as from 80-110 degrees centigrade for 60-120 minutes. As described in the examples below, the reaction can be maintained at 90 degrees centigrade for 90 minutes.

The present disclosure provides certain advantages over previous methods of keratin extraction that have relied on the use of high pH to achieve solubility. Furthermore, these prior methods include the presence of salts in the products of the oxidation process with the subsequent product being not palatable. The disclosed processes differ from previous methods by keeping the pH of the solubilising mixture below the point required for protein hydrolysis. In the second stage of the disclosed processes alkali can be added to raise the pH with care taken to keep the pH <7.5. Any mineral or organic alkali can be used, such as sodium hydroxide, potassium hydroxide or ammonium hydroxide. In contrast to previously described methods in which high pH was required to achieve a satisfactory yield of soluble proteins, the present disclosed processes combine heat, agitation and low pH with oxidation to ensure complete oxidation takes place and the keratin source became soluble. The oxidized mixture is maintained at high temperature for an extended period to ensure complete dissolution. During this step of the process, the solution or suspension is maintained from 80-110 degrees centigrade for 20-90 minutes, or it can be maintained at 90 degrees centigrade for 40 minutes as described in the examples. Any residual oxidant remaining of the process can be removed from the product through the addition of a reductant. Common reductants such as sodium sulfite or sodium metabilsulfite can be used. Following a simple filtration process, the solution of keratin proteins can optionally be further processed with a mass separation method, such as reverse osmosis, nano filtration, dialysis or ultrafiltration to separate residual salts and peptides from the intact protein product. Any commonly known concentration and drying method can be employed to further process the keratin solution into a dry powder. Methods that can be employed include evaporation, spray drying, drum drying and freeze drying. The protein products produced by these methods are both digestible and palatable as a source of nutrition.

An example of a protein powder suitable for a human nutritional supplement was produced as described in example 1. The protein composition was an off white powder with the composition shown in Table 1.

**Table 1 - Keratin Protein Powder**

| Composition | |
|---|---|
| Protein (dry basis) | >87% |
| Moisture | <8% |
| Fat | <5.5% |
| ash | ≤7.5% |
| carbohydrate | <1% |
| Cystine content | >4 residues per 100 |
| Protein Molecular weight | >1000 D |

The processes described produce materials of a high degree of digestibility, high sulfur amino acid content and palatable taste. This is achieved as a result of maintaining a high molecular weight and avoiding the inclusion of peptides and salts in the products that contribute to off taste and odour, whilst still disrupting disulfide bonds associated with keratin insolubility and so making the keratin digestible.

Digestibility of unprocessed keratin sources, such as feathers or wool, is typically low, with in vitro digestibility of approximately 5% commonly reported. Digestibility of the product of example 3 was determined through conventional analysis (AOAC 971.09 2005) as being 89%.

Off tastes in proteins and peptides can be associated with a high degree of salt present in the product, determined as ash in conventional analysis. Typical levels for a keratin hydrolysate are approximately 20-40%. The salt content of the product of example 1 is 6%, determined as ash.

Off tastes can also arise due to the presence of peptides in the product. Peptides typically have a molecular weight of around 1kD or below. By controlling pH to levels lower than that required for hydrolysis, the keratins produced in examples 3 and 4 contain only low levels of peptides. Further, the use of molecular weight separation reduces the amount of peptides in the product. Molecular weight determination using size exclusion chromatography on the product of example 1 and 3 identifies the major components as having a molecular weight above 6.5kD (when compared to aprotinin, a 6.5kD standard used in the analysis), with no substantial peaks associated with peptide material, or material of Mw 1kD or below.

Protein isolates, such as whey or soy protein, are considered to be bland tasting with little off taste or odour. The products of examples 1-4 were compared to soy and whey protein by volunteers in an open label taste comparison. The products of examples 1-4 were all considered to be of similar blandness to soy and whey protein. In particular, the product of example 1 was considered to be indistinguishable from whey protein.

### Example 1

1. Mix 7900ml water, 840ml of 30% (w/w) hydrogen peroxide and heat to 90°C.
2. add 2kg of 'squeezed dry' (typically 35% solids, 65% water) feather (direct from chicken processor) and bring heat back up to 90°C.
3. hold at 90°C for 90 mins, pH typically 3.6 at end of this and feather softened.
4. cool to 60° C then add 100g of 20% (w/w) caustic (NaOH) but keep pH < 7.5 then heat back up to 90°C.
5. Hold at 90°C for 40 mins, add more caustic if pH < 5.9, add 3∼6g caustic to bring pH to 6.1. If it foams, stop adding caustic and reduce stirring. Total caustic addition (including 100g in step 4) is typically 115∼120g (20% (w/w) solution).
6. Remove heat source and allow to cool, filter through 1mm screen.
7. Add 10% (w/w) sodium sulphite solution until there is no residual hydrogen peroxide. Typically 3.5kg is required. Allow 5 minutes of stirring between addition of sodium sulphite solution and assessment of residual hydrogen peroxide level.
8. Cool to 30° C then separate high molecular weight proteins (above ∼ 1kDa) from low molecular weight proteins, peptides and salts using dialysis tubing or ultrafiltration. The retentate conductivity should be typically 1.5 mS at a concentration typically of 5% total solids.
9. Dry and mill to a powder.
   Typical yield 79%

### Example 2

As example 1 except:
In step 1 also add 60 ml of 10% (v/v) H₂SO₄.
In step 4 add 140g of caustic.
In step 5 Total caustic addition will be typically 175g.
   Typical yield 79%

### Example 3

As in example 1 except omit steps 7&8.
Typical yield 99%

### Example 4

As example 1 except omit step 8.
Typical yield 99%

### Example 5 soluble powder for drink or food additive

Keratin powder, prepared according to examples 1-4, was provided for direct addition to food, beverage or water as a supplement. The powder sachet contained 100mg keratin, 100mg vitamin C and 1g maltodextrin.

### Example 6: concentrated keratin liquid supplement

An aqueous solution containing keratin powder, prepared according to example 1 was provided as a concentrated liquid sachet for addition to food, beverage or water. The concentrated liquid contained 5% keratin and a preservative, sodium sulfite.

### Example 7: keratin supplement liquid drink

A 250ml beverage was provided containing the following ingredients

| | |
|---|---|
| Keratin (prepared per example 1) | 2.5g |
| Sucrose | 10g |
| Vitamin C | 1g |
| Colour | <1g |
| Flavour enhancer | <1g |

### Example 8: keratin protein food supplement for athletes

A keratin powder, prepared as per example 1, was formulated to create a protein supplement for athletes. The supplement contained:

| | |
|---|---|
| Keratin (prepared per example 1) | 999g |
| Flavour enhancer (vanilla) | <1g |
| Vitamin supplements | <1g |
| Mineral supplements | <1g |
| Example 9: keratin protein bar | |

Keratin powder, prepared as per example 1, was formulated to create a protein bar for those seeking to boost protein and sulfur intake.

20g of Keratin (prepared as per example 1) was combined with the following ingredients, glucose, milk chocolate, soy lecithin, glycerine, maltodextrin, pineapple juice concentrate, honey, salt and colouring to create a bar of mass 60g with a composition of:

| | |
|---|---|
| Energy | 915kJ |
| Protein | 20g |
| Total fat | 3g |
| Total carbohydrate | 27.3g |
| Example 10 brewed keratin tea drink | |

0.5g Keratin powder, prepared as per example 1 was combined with 0.5g dried green tea leaves and placed in a porous sachet typically used for the preparation of hot tea. The sachet was sealed and immersed in a cup of hot water for 2 minutes to produce a green tea drink with keratin supplementation.

## Claims

1. A nutritional supplement comprising protein powder, wherein the protein powder consists essentially of keratin proteins of greater than 1000 daltons in molecular weight and having a cysteine content of at least 4 per 100 total amino acids, and wherein the powder contains from 85% to 99% protein on a dry weight basis, less than 10% moisture, less than 5% fat, from 1-10% ash, and less than 2% carbohydrate.

2. The nutritional supplement of claim 1, wherein the keratin protein includes less than 5% peptides of less than 1000 daltons in molecular weight and less than or equal to 6% salt.

3. The nutritional supplement of claim 1, wherein essentially all of the cysteine amino acids are oxidized.

4. The nutritional supplement of claim 1 wherein the keratin protein powder is made by a process comprising oxidizing a keratin source in solution at a pH of from 1.5 to 5 and isolating the soluble proteins of molecular weight greater than 1000 daltons.

5. The nutritional supplement of claim 4, wherein the process comprises:
oxidizing by holding a mixture of an insoluble keratin source in an aqueous solution of oxidizing agent at 90°C for 90 minutes;
cooling the mixture to 60° C then adding an amount of base sufficient to raise the pH to produce the protein salt while maintaining a pH below 7.5;
heating the mixture to 90°C and holding for 40 minutes while maintaining the pH below 6.1; and
cooling the mixture and filtering the mixture through a 1 mm screen.

6. The nutritional supplement of claim 5, wherein the process further comprises drying the keratin solution and milling the dried protein to a powder.

7. The nutritional supplement of any preceding claim, further comprising one or more flavorants, one or more sweeteners, one or more vitamins, one or more minerals one or more carbohydrates, or one or more oils.

8. A nutritional supplement in the form of a powder, a bar, or a liquid and comprising a nutritional supplement according to any of claims 1-7.

9. The nutritional supplement of claim 8, which is a brewed liquid comprising tea and keratin protein.

## Patentansprüche

1. Nahrungsergänzungsmittel, das Proteinpulver umfasst, wobei das Proteinpulver im Wesentlichen aus Keratinproteinen von mehr als 1000 Dalton Molekulargewicht und mit einem Cysteingehalt von mindestens 4 pro 100 Gesamt-Aminosäuren besteht und wobei das Pulver von 85% bis 99% Protein auf Trockengewichtsbasis, weniger als 10% Feuchtigkeit, weniger als 5% Fett, von 1 bis 10% Asche und weniger als 2% Kohlenhydrate enthält.

2. Nahrungsergänzungsmittel gemäß Anspruch 1, wobei das Keratinprotein weniger als 5% Peptide von weniger als 1000 Dalton Molekulargewicht und weniger als oder gleich 6% Salz aufweist.

3. Nahrungsergänzungsmittel gemäß Anspruch 1, wobei im Wesentlichen alle Cystein-Aminosäuren oxidiert sind.

4. Nahrungsergänzungsmittel gemäß Anspruch 1, wobei das Keratinproteinpulver durch ein Verfahren hergestellt ist, das ein Oxidieren einer Keratinquelle in Lösung bei einem pH-Wert von 1,5 bis 5 und ein Isolieren der löslichen Proteine mit einem Molekulargewicht von mehr als 1000 Dalton umfasst.

5. Nahrungsergänzungsmittel gemäß Anspruch 4, wobei das Verfahren umfasst:
Oxidieren durch Halten eines Gemisches einer unlöslichen Keratinquelle in einer wässrigen Lösung von Oxidationsmittel bei 90 °C für 90 Minuten;
Kühlen des Gemisches auf 60 °C mit nachfolgendem Zusetzen einer Basenmenge, die ausreicht, um den pH-Wert zu erhöhen, um das Proteinsalz zu erzeugen, während ein pH-Wert unter 7,5 gehalten wird;
Erhitzen des Gemisches auf 90 °C und Halten für 40 Minuten, während der pH-Wert unter 6,1 gehalten wird; und
Kühlen des Gemisches und Filtern des Gemisches durch ein 1-mm-Sieb.

6. Nahrungsergänzungsmittel gemäß Anspruch 5, wobei das Verfahren weiterhin ein Trocknen der Keratinlösung und Mahlen des getrockneten Proteins zu einem Pulver umfasst.

7. Nahrungsergänzungsmittel gemäß einem der vorstehenden Ansprüche, das weiterhin einen oder mehrere Geschmacksstoffe, ein oder mehrere Süßmittel, ein oder mehrere Vitamine, ein oder mehrere Mineralien, ein oder mehrere Kohlenhydrate oder ein oder mehrere Öle umfasst.

8. Nahrungsergänzungsmittel in Form eines Pulvers, eines Riegels oder einer Flüssigkeit, und ein Nahrungsergänzungsmittel gemäß einem der Ansprüche 1 - 7 umfassend.

9. Nahrungsergänzungsmittel gemäß Anspruch 8, das eine aufgebrühte Flüssigkeit ist, die Tee und Keratinprotein umfasst.

## Revendications

1. Complément nutritionnel comprenant une poudre de protéine, cette poudre de protéine consistant essentiellement en des protéines de kératine ayant un poids moléculaire supérieur à 1000 daltons et ayant une teneur en cystéine d'au moins 4 pour 100 de la teneur totale en acides aminés, la poudre renfermant entre 85 % et 99 % de protéines par rapport à son poids sec, moins de 10 % d'humidité, moins de 5 % de graisses, de 1 à 10 % de cendres et moins de 2 % d'hydrates de carbone.

2. Complément nutritionnel conforme à la revendication 1, dans lequel la protéine de kératine renferme moins de 5 % de peptides de poids moléculaire inférieur à 1000 daltons et une quantité de sel inférieure ou égale à 6 %.

3. Complément nutritionnel conforme à la revendication 1, dans lequel essentiellement la totalité des acides aminés de cystéine sont oxydées.

4. Complément nutritionnel conforme à la revendication 1, dans lequel la poudre de protéine de kératine est obtenue par un procédé comprenant des étapes d'oxydation d'une source de kératine dans une solution ayant un pH situé dans la plage de 1,5 à 5 et d'isolement des protéines solubles ayant un poids moléculaire supérieur à 1000 daltons.

5. Complément nutritionnel conforme à la revendication 4, dans lequel le procédé comprend des étapes consistant à :
effectuer une oxydation en maintenant un mélange d'une source de kératine insoluble dans une solution aqueuse d'un agent oxydant à 90°C pendant 90 minutes,
refroidir le mélange à 60°C et ajouter une quantité de base suffisante pour augmenter le pH de façon à obtenir le sel de protéine tout en maintenant le pH au-dessous de 7,5,
chauffer le mélange à 90°C et le maintenir pendant 40 minutes en maintenant le pH en-dessous de 6,1, et
refroidir le mélange et filtrer celui-ci au travers d'un tamis de 1 mm.

6. Complément nutritionnel conforme à la revendication 5, dans lequel le procédé comprend en outre des étapes consistant à sécher la solution de kératine et à broyer la protéine séchée pour obtenir une poudre.

7. Complément nutritionnel conforme à l'une quelconque des revendications précédentes, comprenant en outre au moins un agent aromatisant au moins un agent édulcorant, au moins une vitamine, un ou plusieurs minéraux au moins un hydrate de carbone ou au moins une huile.

8. Complément nutritionnel se présentant sous la forme d'une poudre, d'une barre ou d'un liquide et comprenant un complément nutritionnel conforme à l'une quelconque des revendications 1 à 7.

9. Complément nutritionnel conforme à la revendication 8, constitué par un liquide infusé renfermant du thé et une protéine de kératine.
